# EUROPEAN PATENT APPLICATION

(11) **EP 1 584 341 A1**
(43) Date of publication of application: **12.10.2005**
(21) Application number: 05102686.2
(22) Date of filing: 05.04.2005
(51) Int. Cl.: A61M 5/148, A61M 5/44

(54) **Kit for injecting thermally-conditioned fluids, particularly for first-aid actions**

(30) Priority: 08.04.2004 IT MO20040074
(71) Applicant: Sidam di Azzolini Graziano E C. S.a.s., (Prov.of Modena) (IT)
(72) Inventor: AZZOLINI, Graziano, 41032, CAVEZZO MO (IT); ERCOLANI, Simone Pietro, 06125, PERUGIA (IT); CONTI, Paolo, 56123, PISA (IT)
(74) Representative: Modiano, Guido

(57) **Abstract**

A kit for injecting thermally-conditioned fluids, particularly for first-aid actions, comprising a bag (2) with at least one first pocket (3) therein for containing at least one pouch (5) of fluid to be injected or at least one pouch (5) of fluid to be injected and a pouch-shaped thermal reactor (6), the pocket (3) being provided with a closure flap (7) suitable to contain leaks of the fluid and/or of reagents of the reactor (6), and at least one second pocket (4) for containing an air-filled chamber (8) associated with an air pump (9), at least the first pocket (3) and the flap (7) being made of impermeable and thermally-insulating material.

## Description

The present invention relates to a kit for injecting thermally-conditioned fluids, particularly for first-aid actions.

The present invention is used particularly but not exclusively in first aid to individuals affected by a sudden illness or accidents or injuries directly at the site of the illness, accident or injury, or during their transport toward an equipped health-care facility, such as for example a surgery or a hospital.

Merely by way of example, the present invention is used in first aid provided for work injuries, injuries occurring in locations that are difficult to reach, such as for example the mountains, for road accidents, natural calamities or for terrorist attacks.

It is known that individuals struck by a sudden illness or accident or injury are often in a state of shock characterized by hypovolemia (blood volume reduction) and hypothermia, and require the infusion of fluids at a temperature comprised between 33° and 40° C.

In case of brain-affecting events, it is instead appropriate to reduce the body temperature by infusing chilled fluids.

One of the first treatments that must be provided to patients suffering from hypothermia and/or hypovolemia, therefore, consists in injecting into them fluids, such as for example physiological solutions, plasma substitutes or the like, at a flow-rate and a temperature that allow them to restore normovolemia and normothermia.

The temperature and the quantity of the injected fluid and the time, i.e., the injection rate, are decisive factors on which the rescue of these patients depends; for example, if the temperature of the injected fluid is lower than the normal average body temperature and/or is injected rapidly, the condition of the patient worsens instead of improving.

The fluids to be injected are currently packaged in containers, such as flexible pouches or bottles, which have a dispensing outlet with which the input end of a withdrawal and conveyance duct is associated, the output end of said duct being associated with an element (catheter or needle) for injection into the patient.

To facilitate the discharge of the fluid from the container to the patient, it is known to arrange and keep the container of fluid at a higher level than the patient; the consequent increase in potential energy is converted, for an equal cross-section of the withdrawal and conveyance duct and minus any losses, into an increase in the discharge rate of the fluid.

In first aid provided directly on-site, generally a health worker manually supports the container of fluid, keeping it at a higher level than the patient, whereas in first aid provided during the transport of the patient to an equipped health-care facility the container is hung from the walls or ceiling of the transport vehicle (ambulance, helicopter, et cetera).

However, this method has some drawbacks, including the fact that if a health worker is supporting the container, this limits his maneuvering ability, since at least one hand is busy, preventing him from performing any other first-aid procedures that may be necessary; this drawback is even more onerous if the container is constituted by a pouch, because in this case, in order to increase the discharge of the fluid the operator in fact also squeezes said pouch manually, using both hands and being unable to perform any other maneuver.

Another drawback of this method is that the difference in level between the container and the patient is often insufficient to impart to the fluid a chosen flow-rate and therefore a chosen injection rate.

Another drawback of this method is that the containers, and therefore the fluids contained therein, as well as the duct for withdrawing and conveying the fluid from the container to the patient, known as infusion set in the jargon, by being exposed to the environmental conditions of the site where first aid is performed, may reach an average temperature that is lower than the normal body temperature, causing or increasing hypothermia of the patient.

In order to obviate the drawbacks noted above, various devices for heating the containers and infusion sets of the fluids to be injected and devices, termed "infusion pressor" in the field, are known which are suitable to accelerate the flow of the fluid from the container, constituted by a flexible pouch, to the patient.

In particular, devices for heating the containers of fluid are known which are substantially constituted by boxes that are thermally insulated with respect to the environment that surrounds them and inside which the containers to be conditioned are arranged; said containers are provided with means for generating heat, based for example on electric resistors or microwave radiation, and are provided with electronic circuits for controlling and regulating the temperature.

Devices for heating the duct for withdrawing and conveying the fluid from the container to the patient are also known which substantially consist of a metallic element that is coupled to electric resistors supplied with current and is arranged proximate to the portion of duct to be conditioned; in this case also, an electronic circuit controls and regulates the temperature that is reached.

However, these known heating devices are not free from drawbacks, including the fact that their weight and dimensions are not negligible and make them particularly difficult and onerous to carry onto rescue sites, that they are structurally and constructively complicated, they require the intervention of specialized workers, and have substantial costs.

Another drawback of these known heating devices is that by using electric power they can cause interference with other electrical/electronic devices that are present for example on the vehicles that transport aided individuals.

Devices for heating fluid containers of the so-called chemical type, in that they utilize the heat produced by an exothermic chemical reaction between two or more reacting substances, are also known.

These chemical heating devices are substantially constituted by an enclosure, such as a pouch, which is divided into at least two compartments, which are mutually separated by a tearable membrane, one of said compartments containing a first component, the other compartment containing a second component, said components being suitable to react with each other with an exothermic reaction.

The reaction is usually a reaction of solution of the first component (solute) in the second component (solvent).

The first component is generally in the form of solid particles, granules or the like, and is constituted for example by calcium chloride or the like; the second component is generally in the form of a liquid and is constituted for example by water or the like.

At the time of use, an operator acts manually on the enclosure, for example with a compression or squeezing action, applying thereto a pressure that breaks the tearable membrane and thus allows the second component (solvent) to pour from the second compartment into the first compartment, where it reacts with the first component (solute), dissolving it; if the reaction is exothermic, heat is generated.

After activating the reaction, the enclosure is placed manually in contact with the container of the fluid to be administered in order to condition it thermally and in particular to heat it to the intended temperature.

These known chemical heating devices, too, are not free from drawbacks, including the fact that the reaction between the components contained therein can be activated unintentionally and at inappropriate times by compressions, impacts or accidental squeezing to which they may be subjected during storage, movement and handling, which accordingly require particular care and attention.

These chemical heating devices therefore can exhaust prematurely their ability to generate heat, losing their function and becoming unusable when they are actually needed.

Another drawback of chemical devices is that they contain air, which by having a low heat conductivity acts as an insulator and reduces the exchange of heat with the environment outside them and in particular with the containers of the fluids to be conditioned.

Moreover, the presence of air causes another drawback: it in fact prevents the second component, the one in the liquid state, from diffusing and distributing throughout the entire volume of the first compartment that contains the first component, the one in solid particle or granular form; this reduces the reaction surface, and the reaction remains incomplete, with a consequent decrease in the efficiency of the device.

Known infusion pressors are substantially constituted by a sort of centrally-folding case, inside which a pouch of fluid to be injected is fixed, optionally together with a heating pouch that is one of the chemical heating devices described above; an air-filled chamber is formed within the walls of the case and is connected to manual pumping systems.

The pressure applied by the air pumped into the chamber applies to the pouch of fluid a compression that facilitates the discharge of the fluid contained therein.

However, these known infusion pressors have drawbacks, including the fact that since the folding case is open at least along two opposite sides they do not allow to provide thermal insulation of the pouch of fluid and optionally of the heating pouch with respect to the environment that surrounds them, accordingly exposing them to unpredictable temperature variations, and do not allow to contain any leakage of fluid or heating solution, which might occur if the respective pouches break; these leaks are therefore dispersed into the outside environment and can for example damage other devices or instruments, such as for example the ones that are present in the cabins of rescue vehicles.

Another drawback of known infusion pressors is that they do not allow to check the temperature of the fluid that is gradually injected, with the risk, if said temperature is lower than the normal body temperature, of causing or worsening the hypothermia of the patient.

Another drawback of known infusion pressors is that their transport, handling and support are very difficult and require the use of one or both hands of a health worker, whose possibility to perform other aid maneuvers is therefore impaired if not eliminated.

The aim of the present invention is to eliminate the drawbacks noted above, by providing a kit for injecting thermally-conditioned fluids, particularly for first-aid actions, which allows to inject a fluid in a quantity, at a rate and at a temperature that can be controlled, assisting the return to normovolemia and normothermia of patients.

Another obj ect of the present invention is to provide a kit that allows to improve the heating of the containers of fluid with chemical devices, increasing their efficiency and their heat exchange capacity, facilitating the distribution and diffusion of the reacting components within each other and increasing their reaction surface.

Another obj ect of the present invention is to provide a kit that allows to improve the functionality and safety of chemical heating devices, allowing them to be activated only intentionally, without requiring particular precautions for their storage, movement and handling.

Another object of the present invention is to provide a kit that is structurally and constructively simple, is compact and lightweight, and is easy to transport, handle and use even for less expert operators, giving them ample freedom of maneuver for performing aid procedures.

Within this aim, another obj ect of the present invention is to provide a structure that is simple, relatively easy to provide in practice, safe in use, effective in operation, and has a relatively low cost.

This aim and these and other objects that will become better apparent hereinafter are achieved by the present kit for injecting thermally-conditioned fluids, particularly for first-aid actions, characterized in that it comprises a bag in which there is at least one first pocket for containing at least one pouch of fluid to be injected or at least one pouch of fluid to be injected and a pouch-shaped thermal reactor, said pocket being provided with a closure flap that is suitable to contain any leakage of said fluid and/or reagents of said reactor, and at least one second pocket for containing an air-filled chamber associated with air pumping means, at least said first pocket and said flap being made of impermeable and thermally-insulating material.

Further characteristics and advantages of the present invention will become better apparent from the following detailed description of a preferred but not exclusive embodiment of a kit for injecting thermally-conditioned fluids, particularly for first-aid actions, illustrated by way of non-limiting example in the accompanying drawings, wherein:

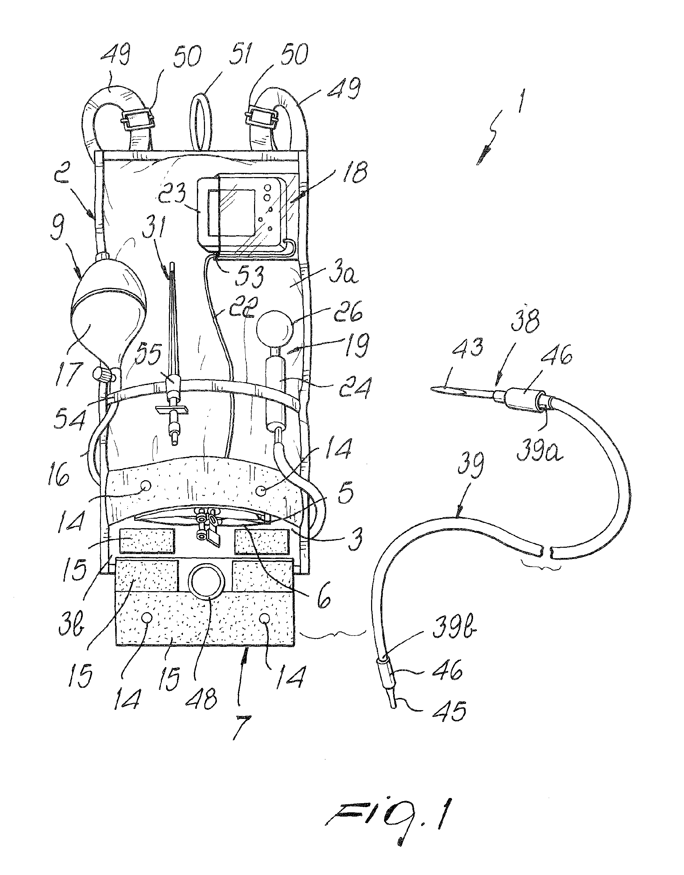

Figure 1 is a schematic view of a kit according to the invention, with the first pocket open;

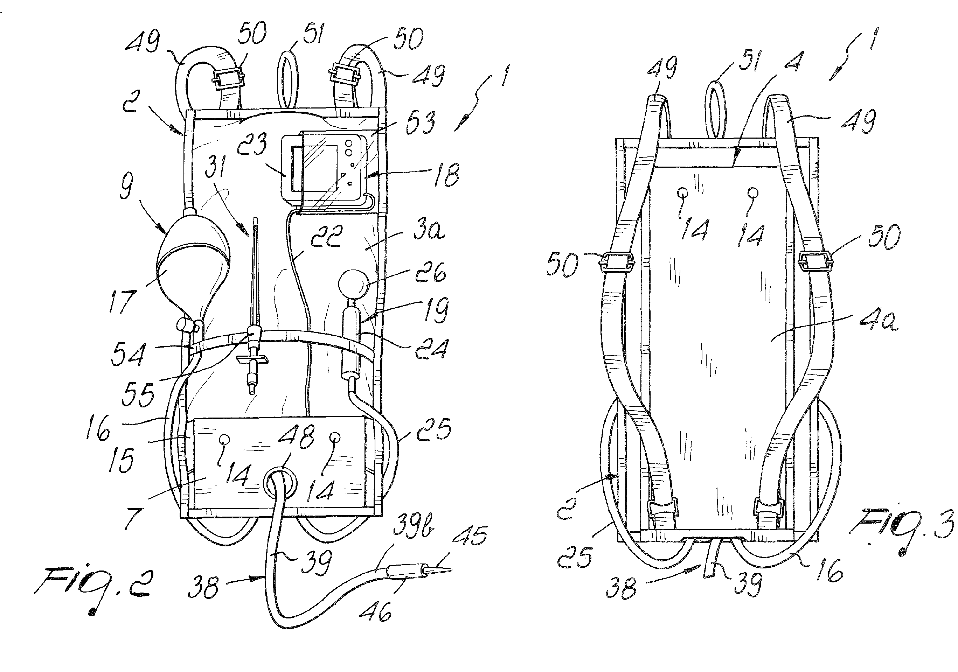

Figure 2 is a schematic front view of the kit according to the invention, in the configuration for use;
Figure 3 is a schematic rear view of the kit of Figure 2;

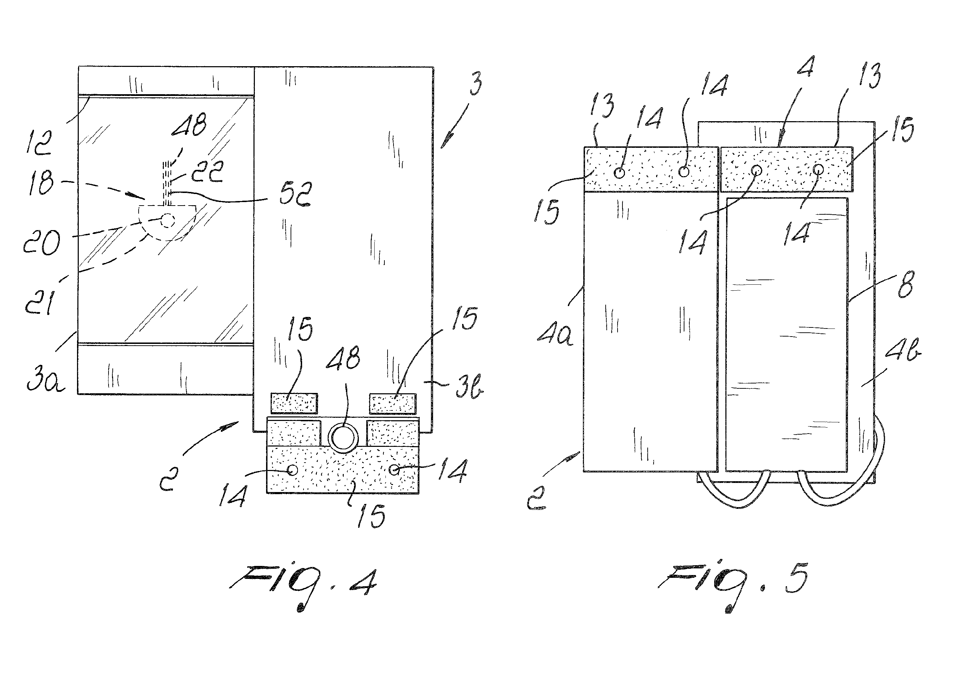

Figure 4 is a schematic flat projection view of the first pocket of the bag of the kit according to the invention;
Figure 5 is a schematic flat projection view of the second pocket of the bag of the kit according to the invention;

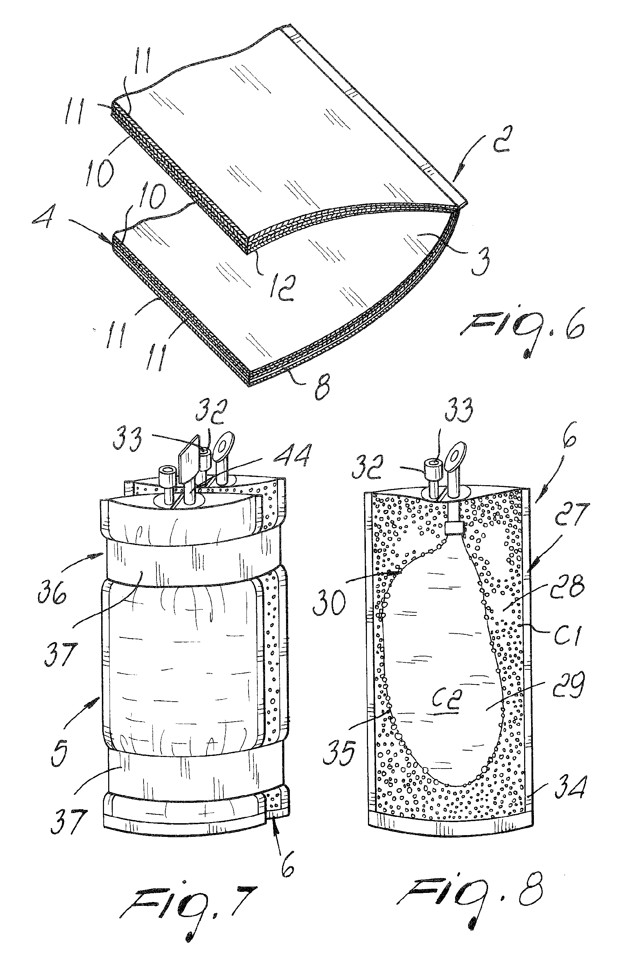

Figure 6 is a partial cutout view of the bag of the kit according to the invention;

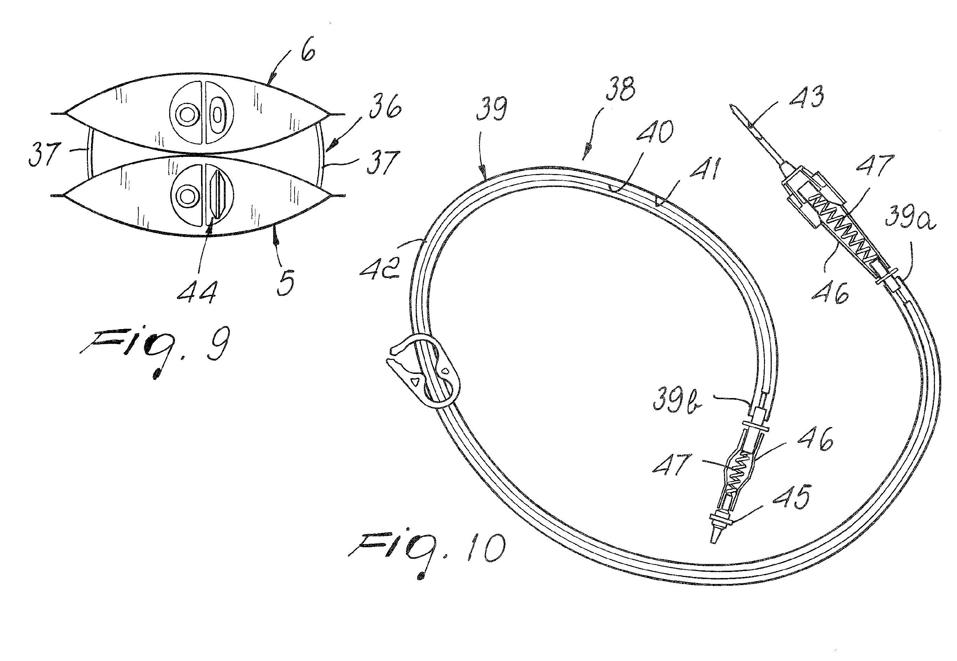

Figure 7 is a schematic view of the thermal reactor coupled to the pouch of fluid of the kit according to the invention;
Figure 8 is a schematic view of the thermal reactor of the kit according to the invention;
Figure 9 is a schematic plan view of Figure 7;
Figure 10 is a schematic enlarged-scale view of the fluid withdrawal and conveyance means of the kit according to the invention.

With reference to the figures, the reference numeral 1 generally designates a kit for injecting thermally-conditioned fluids, particularly for first-aid actions.

The kit 1 comprises a bag 2, provided with a first pocket 3 and a second pocket 4, which substantially mutually overlap.

The first pocket 3 is suitable to contain a pouch 5 of fluid to be injected and optionally also a thermal reactor 6, which is also shaped like a pouch; said first pocket is also provided with a closure flap 7, which is suitable to contain leaks of the fluid contained in the pouch 5 and/or of the reagents of the thermal reactor 6.

The second pocket 4 is suitable to contain an air-filled chamber 8, which is associated with air pumping means 9.

The first pocket 3 and the flap 7 are made of waterproof and thermally-insulating material, constituted by the overlap of a waterproof layer 10 and of one or more thermally-insulating layers 11 made of Nylon (registered trademark), neoprene or the like.

The outer wall 3a of the first pocket 3, directed away from the second pocket 4, is reinforced with a layer of inextensible material 12.

At the flap 7, at the outer wall 3a and inner wall 3b of the first pocket 3 onto which it is folded, and at the end portions 13 of the opposite outer and inner walls 4a and 4b of the second pocket 4 there are closure means, such as press-studs 14, tear-open cling strips 15, or the like.

The outer and inner walls 3a and 3b of the first pocket 3 and the outer and inner walls 4a and 4b of the second pocket 4 are stitched together along three consecutive sides except respectively for the side at which the flap 7 is formed and for the side on which the end portions 13 are formed; these last sides are open for the insertion and extraction respectively of the pouch 5 and/or of the reactor 6 and of the air-filled chamber 8, and can be closed respectively by means of the flap 7 and of the closure means (press-studs 14 and tear-open cling strips 15).

The pumping means 9 comprise a tube 16, which has an end that is associated with the air-filled chamber 8 and an opposite end that is associated with a bulb 17 made of rubber or the like.

The kit 1 further comprises first means 18 for sensing and indicating the temperature of the pouch 5, which is placed in contact with the layer of inextensible material 12, and second means 19 for sensing and indicating the pressure of the air fed into the air-filled chamber 8.

The first sensing and indication means 18 comprise a temperature probe 20, which is arranged inside the first pocket 3 and in particular is coupled, by means of a plate 21, to the layer of inextensible material 12 and is associated by means of a cable 22 with first display means 23 for indicating the detected value.

The second sensing and indication means 19 comprise a pressure gauge 24, which is connected to the air-filled chamber 8 by means of a tube 25 and is associated with second display means 26 for indicating the detected value.

The first and second means for indicating the detected value 23 and 26 comprise a display and/or a graduated scale and/or acoustic warnings and/or luminous warnings, which indicate in particular the exceeding of presettable minimum and/or maximum threshold values respectively of the temperature reached by the pouch 5 and of the pressure inside the air-filled chamber 8.

The reactor 6 is constituted by two surfaces, a thermally-conducting one, which is arranged in contact with the pouch 5, and another surface that is arranged in contact with the internal wall 3b, this arrangement of the reactor 6 being essential in order to detect the temperature of the pouch 5.

The reactor 6 comprises an enclosure 27, which is closed and divided into a first compartment 28, which contains a first component C1, and into a second compartment 29, which contains a second component C2 suitable to react with the first component with an exothermic or endothermic reaction and is separated from the first compartment 28 by a tearable membrane 30, at least one of the first and second compartments 28 and 29 respectively containing the first and second components C1 and C2 substantially in vacuum.

The reactor 6 further comprises means for inserting a tool 31 for tearing the membrane 30: said insertion means are constituted by an inlet 32 and are provided with retention and sealing means, which are suitable to prevent the outflow of the first and second components C1 and C2 and the inflow of the air that is present outside the enclosure 27 and are constituted for example by an insert 33 made of elastic/elastomeric material, which is accommodated in the inlet 32 and is suitable to be crossed by the tool 31 and to close the opening formed therein by said tool.

The first component C1 is in the form of a loose solid, such as particles, granules or the like, and is for example suitable to dissolve with an exothermic reaction in the second component C2, which is in fluid form.

The first component C1 can be constituted for example by calcium chloride and the second component C2 can be constituted for example by water; however, alternative embodiments are not excluded in which the first component C1 and the second component C2 have a different chemical nature.

The enclosure 27 comprises a first bag 34 or the like, the interior of which forms the first compartment 28, and a second bag 35 or the like, which has walls at least partially constituted by the membrane 30 and is accommodated within the first bag 34, its inside forming the second compartment 29.

The first and second bags 34 and 35 are made of a material of the flexible type and are mutually associated proximate to a portion of their walls at which the inlet 32 is formed.

The enclosure 27 and more specifically the first bag 34 comprises at least one heat-conducting portion, which is suitable to be placed in contact with the pouch 5.

Advantageously, the reactor 6 is coupled to the pouch 5 by way of anchoring means 36; in particular, it is arranged so as to overlap the pouch 5 with the heat-conducting portion of the enclosure 27 (first bag 34) in contact with said pouch.

The anchoring means 36 are for example of the type of adhesive tapes 37, straps or the like; as an alternative, the reactor 6 and the pouch 5 can be packaged so as to mutually overlap inside a single containment bag, which is not shown. The tool 31 is of the type of a needle or the like.

Further, the kit 1 comprises withdrawal and conveyance means 38 for withdrawing and conveying the fluid from the pouch 5 to a patient.

The withdrawal and conveyance means 38 comprise a duct 39, which is constituted by a first tubular element 40 for the flow of the fluid and by a second tubular element 41 that provides a sheath and has a substantially larger diameter than the first element, said elements being inserted in each other so as to be substantially coaxial, the gap 42 formed between them being suitable to act as thermal insulation.

A piercing element 43 is associated with the inlet end 39a of the duct 39 and can be inserted in a coupling 44 of the pouch 5 after removing the associated protection seal, while at the outlet end 39b of the duct 39 there is an associated injection element 45, such as a needle, catheter or the like, for injection into the patient.

The inlet end 39a and the outlet end 39b of the duct 9 are provided with respective stiffening sleeves 46 reinforced by a spiral reinforcement 47 that is suitable to avoid kinking.

Conveniently, the display and/or graduated scale of the first and second indicator means 23 and 26, respectively for the sensed temperature and pressure and/or the duct 39 and/or the sleeves 46 and/or their reinforcement 47 are made of a material that is visible even in conditions of poor ambient visibility, such as fluorescent or bright material or the like.

The bag 2 comprises loops 48 for the passage of the cable 22 of the temperature probe 20 and of the duct 39, said loops being formed on the outer wall 3a of the first pocket 3 and/or in the flap 7.

Further, the bag 2 is provided with one or two shoulder straps 49, which are provided with adjusting means 50 for adjusting their length, and with coupling means for coupling to an element for hanging, such as a slot 51 formed at its top.

Finally, the bag 2 comprises containment/anchoring means for containing and/or anchoring the first and second sensing and indication means 18 and 19 and the tool 31, which can be constituted for example by compartments 52 (for the cable 22), cases 53, which are advantageously transparent (for the first indicator means 23), straps 54 or elastic rings 55 (for the tube 16, the pressure gauge 24, the tool 31).

In practice it has been found that the described invention achieves the intended aim and objects.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

The disclosures in Italian Patent Application No. MO2004A000074 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A kit for injecting thermally-conditioned fluids, particularly for first-aid actions, **characterized in that** it comprises a bag (2) in which at least one first pocket (3) is provided for containing at least one pouch (5) of fluid to be injected or at least one pouch (5) of fluid to be injected and a pouch-shaped thermal reactor (6), said pocket (3) being provided with a closure flap (7) that is suitable to retain leaks of said fluid and/or reagents of said reactor (6), and at least one second pocket (4) for containing an air-filled chamber (8) associated with air pumping means (9), at least said first pocket (3) and said flap (7) being made of impermeable and thermally-insulating material.

2. The kit according to claim 1, **characterized in that** said first pocket (3) and said second pocket (4) substantially mutually overlap.

3. The kit according to one or more of the preceding claims, **characterized in that** it comprises first means (18) for sensing and indicating the temperature of said pouch (5) of fluid.

4. The kit according to claim 3, **characterized in that** said first sensing and indication means (18) comprise a temperature probe (20), which is arranged inside said first pocket (3) and is associated by way of a cable (22) with first means (23) for indicating the detected value.

5. The kit according to one or more of the preceding claims, **characterized in that** it comprises second means (19) for sensing and indicating the pressure of the air fed into said chamber (8).

6. The kit according to claim 5, **characterized in that** said second sensing and indication means (19) comprise a pressure gauge (24), which is associated with said air-filled chamber (8) and is associated with second means (26) for indicating the detected value.

7. The kit according to one or more of the claims 3-6, **characterized in that** at least one of said first and second means (23, 26) for indicating the detected value comprises a display and/or a graduated scale and/or acoustic warnings and/or luminous warnings.

8. The kit according to claim 7, **characterized in that** at least one of said display and said graduated scale is made of a material that is visible also in conditions of poor ambient visibility, such as for example fluorescent material, bright material or the like.

9. The kit according to one or more of the preceding claims, **characterized in that** said pumping means (9) comprise a tube (16) that has an end that is associated with said chamber (8) and an opposite end that is associated with a bulb (17) made of rubber or the like.

10. The kit according to one or more of the preceding claims, **characterized in that** it comprises at least one shoulder strap (49), which is provided with means (50) for adjusting its length and is associated with said bag (2).

11. The kit according to one or more of the preceding claims, **characterized in that** it comprises two shoulder straps (49).

12. The kit according to one or more of the preceding claims, **characterized in that** it comprises coupling means (51) for coupling to an element for hanging, coupling means (51) being associated with said bag (2).

13. The kit according to claim 12, **characterized in that** said coupling means comprise a slot (52) formed at the top of said bag (2).

14. The kit according to one or more of the preceding claims, **characterized in that** it comprises closure means (14, 15) that are formed between said flap (7) and said first pocket (3) and/or at the flaps of said second pocket (4).

15. The kit according to claim 14, **characterized in that** said closure means comprise buttons, press-studs (14), tear-open cling strips (15), or the like.

16. The kit according to one or more of the preceding claims, **characterized in that** said material comprises at least one impermeable layer (10) and at least one thermally-insulating layer (11).

17. The kit according to claim 16, **characterized in that** said thermally-insulating layer (11) is made of Nylon, neoprene or the like.

18. The kit according to one or more of the preceding claims, **characterized in that** said first pocket (3) comprises a layer (12) of inextensible material that is formed at its outer wall (3a) that is directed away from said second pocket (4).

19. The kit according to one or more of the preceding claims, **characterized in that** said thermal reactor (6) comprises an enclosure (27) that is closed and divided into at least one first compartment (28), which contains a first component (C1), and into a second compartment (29), which contains a second component (C2) suitable to react with the first component (C1) with an exothermic or endothermic reaction and is separated from said first compartment (28) by a tearable membrane (30), at least one of said first and second compartments (28, 29) containing respectively the first and second components (C1, C2) substantially in vacuum.

20. The kit according to claim 19, **characterized in that** said enclosure (27) comprises means (32) for inserting a tool (31) for tearing said membrane (30).

21. The kit according to claim 20, **characterized in that** said insertion means comprise retention and sealing means (32, 33) that are suitable to prevent the leakage of said first and second components (C1, C2) and the inflow of the air that lies outside said enclosure (27).

22. The kit according to one or more of the claims 20-21, **characterized in that** said insertion means comprise an inlet (32) and said retention and sealing means comprise an insert (33) made of elastic/elastomeric material, which is accommodated in said inlet (32) and is suitable to be crossed by said tool (31) and to close the opening formed therein by said tool (31).

23. The kit according to one or more of the claims 19-22, **characterized in that** at least one (C1) of said first and second components (C1, C2) is in the form of a loose solid, such as particles, granules or the like, the other component (C2) being in fluid form.

24. The kit according to one or more of the claims 19-23, **characterized in that** said enclosure (27) comprises a first bag (34) or the like, the interior of which forms said first compartment (28), and a second bag (35) or the like, the walls of which are at least partially constituted by said membrane (30), said second bag (35) being accommodated inside said first bag (34), its interior forming said second compartment (29).

25. The kit according to one or more of the claims 19-24, **characterized in that** said enclosure (27) is made of a material of the flexible type.

26. The kit according to one or more of the preceding claims, **characterized in that** said enclosure (27) comprises at least one heat-conducting portion.

27. The kit according to one or more of the preceding claims, **characterized in that** said first and second bags (34, 35) are mutually associated at at least one portion of their respective walls.

28. The kit according to claim 27, **characterized in that** said insertion means (32, 33) are associated at said portion.

29. The kit according to one or more of the preceding claims, **characterized in that** said thermal reactor is coupled by way of anchoring means (36) to said pouch (5) of fluid.

30. The kit according to one or more of the claims 19-29, **characterized in that** said enclosure (27) is superimposed on said pouch (5) at least at said heat-conducting portion thereof.

31. The kit according to one or more of the claims 29-30, **characterized in that** said anchoring means (36) are of the type of straps, adhesive tapes (37) or the like.

32. The kit according to one or more of the claims 19-31, **characterized in that** it comprises a tool (31) for tearing said membrane (30).

33. The kit according to one or more of the claims 19-32, **characterized in that** said tearing tool (31) is of the type of a needle or the like.

34. The kit according to one or more of the claims 3-33, **characterized in that** said bag (2) comprises means for containing and/or anchoring said first and second sensing and indication means (18, 19) and said tool (31).

35. The kit according to claim 34, **characterized in that** said containment and/or anchoring means comprise compartments (52), cases (53), straps (54) or elastic rings (55) or the like.

36. The kit according to one or more of the preceding claims, **characterized in that** it comprises means (38) for withdrawing and conveying said fluid from said pouch (5) to a patient.

37. The kit according to claim 36, **characterized in that** said withdrawal and conveyance means (38) comprise a duct (39), which is constituted by a first tubular element (40) for the flow of said fluid and by a second tubular sheathing element (41), which has a substantially larger diameter than the first tubular element (40), said elements (40, 41) being inserted in each other so as to be substantially coaxial, the gap (42) between them being suitable to act as thermal insulation.

38. The kit according to claim 37, **characterized in that** said duct (39) comprises an inlet end (39a), with which it is possible to associate an element (43) for piercing said pouch (5) of fluid, and an outlet end (39b), with which it is possible to associate an element (45) for injection into a patient.

39. The kit according to claim 38, **characterized in that** it comprises stiffening sleeves (46) associated with said inlet and outlet ends (39a, 39b) of said duct (39).

40. The kit according to claim 39, **characterized** that said sleeves (46) comprise a spiral reinforcement (47).

41. The kit according to one or more of the claims 37-40, **characterized in that** said duct (39) and/or said sleeves (46) are made of a material that is visible also in conditions of poor atmospheric visibility, such as bright material, phosphorescent material or the like.

42. The kit according to one or more of the claims 4-41, **characterized in that** it comprises loops (48) for passage of said cable (22) of the temperature probe (20) and of said duct (39), which are formed in said first pocket (3) and/or in said flap (7).
